Europäisches Patentamt

European Patent Office

Office européen des brevets

(11) Numéro de publication: **0 170 302**

**A1**

# DEMANDE DE BREVET EUROPEEN

(21) Numéro de dépôt: **85200746.7**

(22) Date de dépôt: **10.05.85**

(51) Int. Cl.⁴: **G 01 N 33/543**
//G01N33/546, G01N33/577

(30) Priorité: **27.06.84 BE 213225**

(43) Date de publication de la demande:
**05.02.86 Bulletin 86/6**

(84) Etats contractants désignés:
**AT CH DE FR GB IT LI LU NL SE**

(71) Demandeur: **l'Association internationale à but scientifique, dite: Institut international de pathologie cellulaire et moléculaire**
**Avenue Hippocrate 75**
**B-1200 Woluwé-Saint-Lambert(BE)**

(72) Inventeur: **Masson, Pierre**
**Avenue Emile Vandervelde, 107**
**B-1200 Bruxelles(BE)**

(74) Mandataire: **Schmitz, Yvon et al,**
**Bureau Gevers S.A. 7, rue de Livourne Bte 1**
**B-1050 Bruxelles(BE)**

(54) **Procédé de dosage immunologique d'une substance dans un échantillon liquide au moyen d'anticorps anti-idiotypiques.**

(57) Procédé de dosage immunologique d'une substance dans un échantillon liquide, ladite substance comportant au moins un déterminant important pour son identification, comprenant la mise en réaction dudit échantillon liquide contenant la substance à doser, d'anticorps dirigés contre ladite substance, spécifiques dudit déterminant et d'anticorps anti-idiotypiques dirigés contre les anticorps spécifiques et la détermination de la quantité de la substance dans l'échantillon en mesurant le degré d'inhibition qu'entraîne cette substance sur la réaction de liaison entre les anticorps susdits.

EP 0 170 302 A1

"Procédé de dosage immunologique d'une substance dans un échantillon liquide au moyen d'anticorps anti-idiotypiques".

La présente invention est relative à un procédé de dosage immunologique d'une substance dans un échantillon liquide, tel qu'un fluide biologique , cette substance comportant au moins un déterminant important pour son identification.

L'utilisation d'anticorps pour le dosage d'une substance avec une sensibilité et une spécificité très élevées, est bien connue et est la base des radioimmunoessai, enzyimmunoessai, fluoroimmunoessai, immuno-essai par comptage de particules, etc. Dans tous les cas, on injecte un échantillon très pur de la substance à doser chez un animal et, après des injections répétées, on prélève le sang de l'animal, on sépare le sérum et l'on utilise ce dernier comme réactif pour le dosage de la substance. Le sérum contient une classe spéciale de protéines que l'animal a produites, qui se lient d'une manière spécifique à la substance que l'on a injectée et qui, par liaison à la substance, forment des agrégats, que l'on peut séparer par centrifugation, précipitation ou une quelconque des autres méthodes de séparation disponibles. Ces protéines spéciales appartiennent à la classe connue des "immunoglobulines", dont cinq classes principales connues sous la dénomination de "IgG, IgA, IgM, IgE et IgD", qui existent chez la plupart des espèces animales. Seul un faible pourcentage d'immunoglobuline est adapté pour réagir avec la substance à doser. Cette portion est appelée "anticorps". Si l'on désire doser, par exemple, des immunoglobulines de ce type appartenant à l'être humain, il suffit de purifier l'une des immunoglobulines humaines, par exemple l'IgG,d'injecter l'immunoglobuline purifiée chez un autre animal, par exemple un lapin, et en même temps,de séparer un sérum de lapin qui liera cette immunoglobuline humaine spécifique, qui a été injectée initialement chez le lapin. Le sérum du lapin qui réagit avec l'immunoglobuline humaine contiendra probablement des anticorps IgG de lapin et IgM de lapin, qui seront présents en des quantités de l'ordre de 1 à 2% de l'IgG ou IgM total.

Evidemment, les anticorps reconnaissant différentes substances sont différents les uns des autres et cette différence réside dans la disposition chimique d'une partie spécifique de l'immunoglobuline qui est apte à reconnaître la substance à doser. On appelle cette partie le "site de liaison d'antigène", et elle reconnaît une partie spécifique de la substance à doser, c'est-à-dire une petite chaîne de molécules appelée "déterminant". Toutefois, la plupart des substances complexes que l'on doit analyser comportent plus d'une chaîne de molécules caractéristique et comportent,par conséquent, plus d'un déterminant. Dans la population des anticorps produits, il y aura des anticorps qui se fixent à chaque déterminant et, dans la plupart des cas, les anticorps qui se fixent à un déterminant de la substance ne se fixeront pas à un autre déterminant de celle-ci. En résumé, un antisérum contiendra des anticorps d'un grand nombre de types, chaque type d'anticorps s'agglutinant par l'intermédiaire d'un déterminant différent sur la substance et ne constituant qu'une partie des anticorps totaux, qui eux mêmes ne représentent qu'une faible proportion des immunoglobulines totales. Par conséquent, il y aura autant de sites de liaison d'antigène qu'il y a de déterminants.

Si, maintenant, l'on sépare chaque anticorps avec un site de liaison d'antigène donné, par exemple, d'un antisérum de lapin dirigé contre de l'IgG humaine et que l'on injecte l'un de ces anticorps, par exemple, chez une chèvre, la chèvre produira des anticorps qui reconnaîtront les anticorps de lapin. Les anticorps de chèvre réagiront avec les déterminants qui sont communs à l'immunoglobuline de lapin mais certains des anticorps peuvent reconnaître d'une manière spécifique des déterminants localisés dans les sites de liaison d'antigène des anticorps de lapin. Ces déterminants sont habituellement appelés des "idiotopes" et l'association des différents idiotopes d'un anticorps donné est appelée un "idiotype". Les antisérums ou anticorps réagissant avec des idiotypes sont appelés "anti-idiotypiques".

Bien que l'on puisse subdiviser les immuno-essais de plusieurs façons, on les divisera dans le cas présent, pour une question de commodité, en deux types majeurs, à savoir les dosages par agglutination directe et les dosages par inhibition d'agglutination. Dans les dosages par agglutination directe, la substance à doser dans sa forme naturelle est impliquée directement dans l'agglutination. Evidemment, la substance doit comporter plus d'un déterminant. Le procédé d'agglutination implique alors un agglutinat du type anticorps-substance- anticorps-substance-anticorps, etc., la dimension des agglutinats dépendant d'un grand nombre de facteurs. Une fois la réaction terminée, la quantité d'anticorps restant sera inversament proportionnelle à la concentration de la substance initialement présente.

La présente invention ne concerne toutefois que les dosages du second type, c'est-à-dire les dosages par inhibition d'agglutination. Ce second type de dosage s'avère intéressant pour les substances qui ne comportent qu'un seul déterminant ou pour les substances de composition tellement proche qu'elles ne diffèrent que par un seul déterminant important. Par exemple, ce type de dosage s'avère particulièrement intéressant pour des molécules simples, telles que la thyroxine (T4) ou la progestérone ou pour un grand nombre d'autres hormones qui sont trop petites pour comporter plus d'un déterminant et qui ne peuvent pas entrer dans une réaction d'agglutination. D'une autre côté, certaines molécules de grandes dimensions, telles que l'antigène carcinoembryonnaire (CEA), un agent de détection des affections cancéreuses, comporte un grand nombre de déterminants différents mais il n'y a probablement qu'un petit nombre de déterminants sur le CEA qui soient réellement distinctifs,les autres déterminants apparaissant sur des substances analogues qui ne sont pas des agents de détection du cancer.
La présente invention permet d'améliorer les dosages
pour molécules de structure simple et procure un
nouveau moyen permettant de doser les molécules de
structure complexe comportant un ou plusieurs déterminants importants pour l'identification de la molécule à doser, c'est-à-dire donnant l'individualité à
celle-ci.

On décrit ci-après la technique habituellement appliquée au dosage des molécules simples, en
prenant comme exemple la triiodothyronine (T3). On
peut conférer à une substance un grand nombre de déterminants, chaque déterminant étant constitué par
une molécule T3 en liant plusieurs T3 à une macromolécule, telle que du dextran ou de l'albumine. La
substance dextran-T3 devient alors une substance
comportant plusieurs déterminants. Si l'on ajoute
maintenant des anticorps produits par exemple chez une
souris dirigés contre la substance dextran-T3, ces
anticorps formeront des agglutinats : anticorps-dextran-
T3-anticorps-dextran-T3, etc. Si l'on ajoute maintenant du sérum d'un être humain qui contient de la T3 seule
(c'est-à-dire non liée), cette T3 libre se liera au site de liaison
d'antigène des anticorps de souris, et de la sorte ces
anticorps ne pourront plus réagir. Lorsque l'on ajoute
maintenant le dextran-T3, il y aura moins d'anticorps
avec un site de liaison d'antigène libre et l'agglutination sera par conséquent moins importante. La T3
libre dans le sérum humain a par conséquent inhibé
l'agglutination des anticorps dirigés contre la
substance dextran-T3. Cette technique est bien connue et a été décrite dans un grand nombre de brevets antérieurs.

Toutefois, cette technique comporte plusieurs inconvénients, dont les principaux sont les
suivants :

1) La liaison d'une substance simple à
une molécule de plus grande dimension est souvent
coûteuse et difficile, si on veut la réaliser d'une
manière efficace. Bien que l'on puisse faire entrer
ces prix élevés dans le cadre de la fabrication d'une
substance productrice d'anticorps, son utilisation
comme réactif "en vrac" dans un dosage peut fortement
accroître le coût de celui-ci.

2) La sensibilité d'un grand nombre de
dosages est limitée car les anticorps reconnaissent
comme déterminants d'autres groupements que la substance simple, c'est-à-dire la substance simple combinée
à d'autres atomes ou configurations atomiques entourant
celle-ci dans la molécule de grande dimension. Par conséquent, dans les mélanges, tels que celui constitué,
par exemple, de T3, de dextran-T3 et d'anticorps, les
anticorps ont une préférence pour le dextran-T3. Pour
surmonter cette préférence, l'utilisateur est forcé
d'avoir une quantité de T3 plus grande que la quantité
théoriquement requise, c'est-à-dire de travailler avec
une sensibilité réduite.

L'objet de la présente invention consiste,
par conséquent, à pallier les inconvénients susmentionnés
des procédés de dosage connus , à prévoir un procédé de
dosage immunologique , avec une très grande sensibilité et
spécificité, d'une substance dans un échantillon liquide, tel qu'un fluide biologique, cette substance pouvant aussi bien être de structure moléculaire simple, c'est-à-dire comporter un seul déterminant pour son identification, que de structure moléculaire complexe, c'est-à-dire comportant un ou plusieurs déterminants importants pour son identification.

A cet effet, suivant l'invention, on fait réagir l'échantillon liquide contenant la substance à doser, des anticorps dirigés contre ladite substance, spécifiques dudit déterminant et des anticorps anti-idiotypiques dirigés contre les anticorps spécifiques et on détermine la quantité de la substance dans l'échantillon liquide en mesurant le degré d'inhibition qu'entraîne cette substance sur la réaction de liaison entre les anticorps spécifiques et anti-idiotypiques susdits, la quantité de cette substance étant proportionnelle au degré d'inhibition obtenu sur la réaction de liaison précitée.

Suivant une forme de réalisation particulière du procédé de l'invention, soit les anticorps anti-idiotypiques soit les anticorps spécifiques sont fixés à des particules finement divisées, telles que des particules de latex, des globules rouges, et on détermine la quantité de substance dans l'échantillon liquide, après réaction de cette substance et des anticorps spécifiques et anti-idiotypiques précités, en fonction du degré d'agglutination résiduaire ou de non agglutination de ces particules finement divisées.

Suivant une première forme de réalisation particulièrement avantageuse du procédé de l'invention, on accroît l'activité agglutinante des anticorps anti-idiotypiques par l'addition d'un agent susceptible d'accroître une telle activité, choisi,par exemple , dans le groupe comprenant le facteur rhumatoïde, le facteur Clq du complément et le polyéthylèneglycol.

Suivant une autre forme de réalisation particulièrement avantageuse du procédé de l'invention, dans le cas où les anticorps anti-idiotypiques sont fixés à des particules finement divisées, on préincube l'échantillon liquide contenant la substance à doser avec les anticorps spécifiques sous agitation à une température de l'ordre de 37°C pendant une durée de l'ordre de 1 minute à 18 heures.

Le procédé de la présente invention peut, bien entendu, être également appliqué aux techniques de dosage radioimmunologique avec marqueurs, c'est-à-dire dans les techniques qui impliquent l'utilisation d'anticorps marqués par des substances radioactives,enzymatiques ou autres. Ces techniques de dosage immunologique , dont notamment la technique du double-anticorps, comportent également des inconvénients. Cette technique du double-anticorps est actuellement la technique de dosage la plus largement utilisée en pratique.Cette technique consiste à insolubiliser les anticorps dirigés conte les substances à doser en les couplant à une phase solide, qui peut être constituée par des micro-plaques de polyvinyle,des billes d'agarose , des disques de papier, etc. Ces anticorps insolubilisés, lorsqu'ils sont mélangés à l'échantillon à analyser, retiendront la substance. Après avoir écarté les molécules non liées par lavage, la présence de la substance est décelée par la liaison d'anticorps marqués qui reconnaissent les déterminants antigéniques différents de ceux reconnus par les anticorps insolubilisés. Comme on vient de le préciser, les anticorps peuvent être marqués par un radioisotope, une enzyme, ou bien également par un fluorochrome ou des particules colloïdales. Ce marquage permettra de mesurer les anticorps liés, dont la quantité sera proportionnelle à la quantité de substance retenue par les anticorps insolubilisés. Ce type de dosage comporte toutefois deux inconvénients : il ne peut être appliqué qu'à des substances comportant au moins deux déterminants antigéniques, ce qui n'est pas le cas des petites substances telles que la T3 ou la progestérone, et il requiert deux populations d'anticorps reconnaissant des déterminants antigéniques différents. On peut, par exemple, utiliser deux anticorps monoclonaux avec des spécificités différentes. Toutefois, pour certains antigènes, il peut s'avérer difficile d'obtenir des anticorps monoclonaux avec des spécificités qui ne se recouvrent pas. Le procédé de dosage de la présente invention permet de surmonter ces inconvénients grâce à l'utilisation d'anticorps anti-idiotypiques, et de pouvoir donc être utilisé également pour le dosage de substances simples ne comportant qu'un seul déterminant important permettant d'identifier celles-ci.

Par conséquent, suivant une autre forme de réalisation de l'invention, les anticorps anti-idiotypiques sont marqués au moyen d'une substance active choisie dans le groupe comprenant les radioisotopes, les enzymes, les fluorochromes et les particules colloïdales et les anticorps spécifiques
couplés à une phase solide de manière à les insolubiliser, et on ajoute l'échantillon liquide contenant la
substance à doser aux anticorps insolubilisés, on
laisse réagir ces anticorps insolubilisés avec ladite
substance, on ajoute à l'échantillon ainsi obtenu
des anticorps anti-idiotypiques marqués, on laisse
réagir ces anticorps anti-idiotypiques marqués, on
sépare de la phase solide ces anticorps anti-idiotypiques marqués non liés aux anticorps insolubilisés, et on détermine la quantité de substance à
doser dans l'échantillon liquide en fonction de
l'activité résiduaire des anticorps anti-idiotypiques
marqués liés à la phase solide.

Suivant une seconde forme de réalisation
particulière de l'invention, les anticorps spécifiques
sont marqués au moyen d'une substance active choisie
dans le groupe comprenant les radioisotopes, les
enzymes, les fluorochromes et les particules colloïdales
et les anticorps anti-idiotypiques couplés à une
phase solide de manière à les insolubiliser, et on
ajoute l'échantillon liquide contenant la substance
à doser aux anticorps insolubilisés, on laisse réagir
ces anticorps insolubilisés avec ladite substance,
on ajoute à l'échantillon ainsi obtenu les anticorps
spécifiques marqués, on laisse réagir ces anticorps
spécifiques marqués, on sépare de la phase solide les
anticorps spécifiques marqués non liés aux anticorps
insolubilisés et on détermine la quantité de substance
à doser dans l'échantillon liquide en fonction de l'activité résiduaire des anticorps spécifiques marqués liés à la phase solide.

Suivant encore une autre forme de réalisation particulière de l'invention, les anticorps spécifiques sont couplés à une phase solide de manière à les insolubiliser et on ajoute l'échantillon liquide contenant la substance à doser aux anticorps insolubilisés, on laisser réagir ces anticorps insolubilisés avec ladite substance, on ajoute à l'échantillon ainsi obtenu les anticorps anti-idiotypiques, on laisser réagir ces anticorps anti-idiotypiques, on sépare de la phase solide les anticorps anti-idiotypiques non liés aux anticorps insolubilisés, on ajoute à la phase solide des anticorps dirigés contre les anticorps anti-idiotypiques, marqués au moyen d'une substance active choisie dans le groupe comprenant les radioisotopes, les enzymes, les fluorochromes et les particules colloïdales, on laisse réagir ces anticorps marqués, on sépare de la phase solide les anticorps marqués non liés aux anticorps anti-idiotypiques et on détermine la quantité de substance à doser dans l'échantillon liquide en fonction de l'activité résiduaire des anticorps marqués liés à la phase solide.

D'autres détails et particularités de l'invention ressortiront de la description donnée ci-après à titre d'exemple non limitatif de quelques formes de réalisation particulièresde l'invention.

On notera que le procédé de dosage immunologique de la présente invention est en fait facilité par l'utilisation d'anticorps spécifiques, c'est-à-dire d'anticorps monoclonaux dont la production, notamment

12   0170302

chez la souris, est bien connue. La caractéristique
d'un anticorps monoclonal est qu'il se fixe à un
déterminant spécifique sur la substance à doser. Si,
par conséquent, il y a par exemple cinq déterminants
sur une molécule d'hormone de croissance humaine, cinq
anticorps monoclonaux différents pourraient être
induits, un pour chaque déterminant. Bien que chaque
anticorps soit une IgG, son idiotype diffère,
c'est-à-dire qu'il présente effectivement une composition chimique différente dans la région qui reconnaît
le déterminant. Puisque l'anticorps a une composition
chimique différente, il serait alors théoriquement
possible de produire un antisérum contre chaque idiotype en injectant par exemple à un lapin l'anticorps
monoclonal spécifique. L'antisérum de lapin contiendra certainement des anticorps dirigés contre l'IgG
de souris mais parmi ces anticorps il y en aura une
partie qui reconnaîtra l'idiotype d'une manière spécifique, c'est-à-dire que l'antisérum est anti-idiotypique.
Comme le savent bien les spécialistes de la technique,
ces anticorps anti-idiotypiques IgG très spécifiques
peuvent être séparés et utilisés comme réactif.

Par conséquent, dans un dosage immunologique spécifique de la triiodothyronine (T3), si l'on
fait réagir des anticorps anti-T3 monoclonaux de souris
à titre d'anticorps spécifiques avec des anticorps
anti-idiotypiques IgG de lapin dirigés contre les anticorps monoclonaux de souris, à titre d'anticorps anti-
idiotypiques, les anticorps anti-idiotypiques agglutineraient alors les anticorps anti-T3 monoclonaux de
souris. Si, maintenant, l'on avait mélangé l'échantillon liquide contenant la substance à doser, c'est-à-dire dans le cas présent du sérum humain contenant de la T3 avec des anticorps anti-T3 monoclonaux de souris, la triiodothyronine aurait réagi avec les anticorps monoclonaux de souris, en bloquant le centre idiotypique . Par contre, lorsque l'on fait réagir, suivant l'invention, ce mélange de sérum humain contenant la T3 et d'anticorps monoclonaux de souris avec des anticorps anti-idiotypiques de lapin, l'agglutination sera sensiblement moins forte. On déterminera, dans ce cas, la quantité de T3 dans le sérum humain en mesurant le degré d'inhibition qu'entraîne la T3 sur la réaction de liaison entre les anticorps spécifiques et anti-idiotypiques, qui est en fait une réaction d'agglutination, la quantité de T3 étant proportionnelle au degré d'inhibition obtenu sur cette réaction d'agglutination. Dans ce procédé de dosage, il n'est par conséquent plus nécessaire de lier la substance à doser à une macromolécule, de manière à ce qu'elle contienne un grand nombre de déterminants, et il n'y a plus de compétition entre les déterminants comprenant la substance liée et la substance libre.

Ainsi qu'on l'a déjà précisé précédemment, le procédé de dosage de l'invention peut être également étendu au dosage d'une substance de structure moléculaire complexe, comportant un seul ou éventuellement plusieurs déterminants importants pour son identification. Ces déterminants peuvent exister sur les parois des bactéries ou virus, ou constituent même une petite partie d'une molécule complexe. L'isolement de ces fractions ou déterminants spécifiques est actuellement technologique- ment souvent très difficile à réaliser, si pas impossible. Pour les spécialistes en matière de production d'anticorps monoclonaux, la séparation à l'état pur de ces déterminants n'est souvent pas nécessaire pour avoir des anticorps monoclonaux hautement spécifiques. Par exemple, on peut obtenir des anticorps monoclonaux contre le déterminant de l'antigène carcinoembryonnaire (CEA), qui est l'élément distinctif dans la détection des tumeurs cancéreuses. Lorsque l'on injecte ces anticorps monoclonaux, par exemple des anticorps anti-CEA monoclonaux de souris chez un lapin, le lapin produira des anticorps anti-idiotypiques IgG de lapin dirigés contre les anticorps monoclonaux de souris, qui eux mêmes sont dirigés contre le déterminant spécifique de CEA .

En l'absence de CEA, les anticorps anti-idiotypiques de lapin formeront des agglutinats avec les anticorps monoclonaux de souris. Toutefois, lorsque l'on mélange les anticorps monoclonaux de souris avec, par exemple, un sérum humain contenant du CEA, le CEA réagira avec l'idiotype monoclonal et bloquera alors toute agglutination ultérieure entre les anticorps anti-idiotypiques de lapin et les anticorps monoclonaux de souris. Le degré de cette inhibitioin d'agglutination sera proportionnelle à la concentration de CEA dans le fluide biologique.

Suivant l'invention, la mise en réaction ou le mélange entre l'échantillon liquide contenant la substance à doser, les anticorps spécifiques et les anticorps anti-idiotypiques comprend, d'une manière générale, l'incubation de cet échantillon liquide et de ces anticorps sous agitation à une température d'environ 37°C pendant une durée très variable suivant le type de dosage ou de substance utilisé , pouvant aller par exemple de l'ordre de 15 minutes à 24 heures.

On notera également, suivant l'invention, que l'on obtiendra une meilleure sensibilité dans le dosage immunologique de la substance, dans le cas où les anticorps anti-idiotypiques sont fixés à des particules finements divisées, en préincubant l'échantillon liquide contenant la substance à doser avec les anticorps spécifiques sous agitation à une température de l'ordre de 37°C, pendant une durée de l'ordre de 1 minute à 18 heures, qui dépend principalement du type de la substance à doser. Dans le cas où l'on utilise des anticorps marqués, on préincubera l'échantillon liquide contenant la substance à doser avec ceux-ci à une température et pendant une durée similaires à celles qui viennent d'être citées.

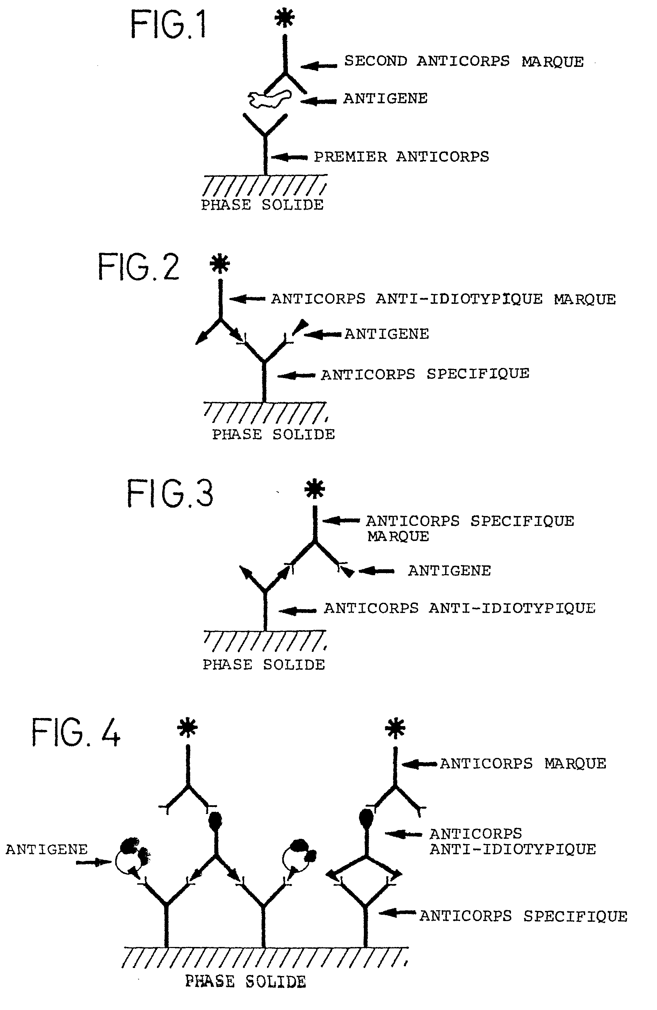

Ainsi qu'on l'a déjà mentionné précédemment, le procédé de la présente invention peut être appliqué dans le cadre des techniques de radioimmunoessai, c'est-à-dire en utilisant des anticorps marqués, tout en ne présentant pas les inconvénients que ces techniques de radioimmunoessai actuelles comportent, telles que la technique du double anticorps, c'est-à-dire le fait qu'elles ne peuvent être appliquées qu'à des substances comportant au moins deux déterminants antigéniques et d'obtenir des anticorps monoclonaux dont les spécificités ne se recouvrent pas. On se référera, à cet effet, en ce qui concerne la technique du double anticorps, à la figure 1 des dessins annexés.

Comme on l'a déjà expliqué, le premier anticorps est
fixé à la phase solide et le second anticorps est marqué au moyen d'un radioisotope, d'une enzyme, d'un
fluorochrome ou d'une particule colloïdale. Suivant
l'invention, l'utilisation d'anticorps anti-idiotypiques marqués, tel qu'on peut le voir sur la figure 2
des dessins annexés, permet de surmonter la limitation
des techniques radioimmunologiques connues, notamment
celle du double anticorps, et de permettre le dosage
de substances de structure moléculaire simple, telles
que la T3 ou la progestérone. Comme on peut le voir
sur cette figure 2, les anticorps anti-idiotypiques
sont marqués par une substance active du type radioisotope, enzyme, fluorochrome ou particule colloïdale
et les anticorps spécifiques sont couplés à une phase
solide qui peut être constituée, par exemple, d'un
support polymère, tel que microplaques de chlorure de
polyvinyle, billes d'agarose, disques de papier ou de
cellulose. On ajoute ensuite l'échantillon liquide
contenant la substance ou l'antigène à doser aux
anticorps insolubilisés, on laisse réagir ces anticorps
insolubilisés avec l'antigène, on ajoute à l'échantillon
ainsi obtenu les anticorps anti-idiotypiques marqués,
on laisse réagir ces anticorps anti-idiotypiques marqués, on sépare de la phase solide ces anticorps anti-
idiotypiques marqués non liés aux anticorps insolubilisés
et on détermine la quantité de substance antigénique
à doser dans l'échantillon liquide en fonction de
l'activité résiduaire des anticorps anti-idiotypiques
marqués liés à la phase solide. En fait, la liaison
de l'antigène aux anticorps empêchera la liaison des anticorps anti-idiotypiques marqués aux anticorps insolubilisés ( essai d'inhibition). Par conséquent, le signal donné par le marqueur diminuera avec l'augmentation de la quantité d'antigène.

Une autre forme de réalisation suivant l'invention consiste à utiliser des anticorps anti-idiotypiques insolubilisés, les anticorps marqués étant alors les anticorps dirigés contre l'antigène ou la substance à doser, comme on peut le voir sur la figure 3. On procède d'une manière analogue aux cas précédents mais on sépare, bien entendu, de la phase solide les anticorps spécifiques marqués non liés aux anticorps insolubilisés et on détermine la quantité de substance antigénique à doser dans l'échantillon liquide en fonction de l'activité résiduaire des anticorps spécifiques marqués liés à la phase solide.

Une troisième possibilité, suivant l'invention, est d'utiliser un troisième type d'anticorps, ces anticorps constituant alors les anticorps marqués. Le principe de ce dosage, finalement très similaire aux deux cas précédents, est représenté à la figure 4. On couple, en fait, dans ce cas, les anticorps spécifiques à la phase solide et on ajoute l'échantillon liquide contenant l'antigène ou la substance à doser aux anticorps ainsi insolubilisés, on laisse réagir ces anticorps insolubilisés avec la substance antigénique, on ajoute à l'échantillon ainsi obtenu des anticorps anti-idiotypiques, on laisse réagir ces anticorps anti-idiotypiques, on sépare de la phase solide les anticorps anti-idiotypiques non liés aux anticorps insolubilisés, on ajoute à la phase solide les anticorps marqués, dirigés contre les anticorps anti-idiotypiques, on laisse réagir ces anticorps marqués, qui reconnaissent en fait les immunoglobulines auxquelles appartiennent les anticorps anti-idiotypiques, on sépare de la phase solide les anticorps marqués non liés aux anticorps anti-idiotypiques et on détermine la quantité de substance antigénique à doser dans l'échantillon liquide en fonction de l'activité résiduaire des anticorps marqués liés à la phase solide, cette phase solide pouvant également être constituée, comme dans les deux cas précédents, d'un support polymère, tel que microplaques de polyvinyle, billes d'agarose, disques de papier, etc. Dans les trois formes de réalisation susmentionnées, la séparation des anticorps non liés de la phase solide se fait généralement par lavage dans une solution saline, ou encore par simple égouttage, décantage, précipitation ou centrifugation. Ce procédé convient particulièrement bien au dosage de molécules complexes, telles que les allergènes.

Les trois exemples concrets donnés ci-après permettent d'illustrer davantage le procédé suivant l'invention.

Exemple 1.

Dosage d'immunoglobuline IgE (molécule complexe) en utilisant la technique d'immunoessai par comptage de particules (PACIA).

Vingt-sept anticorps monoclonaux différents de souris sont dirigés contre la molécule IgE à doser dans des sérums humains et l'on choisit l'un de ces anticorps monoclonaux, que l'on appellera ci-après B9. Les anticorps monoclonaux B9 réagissent d'une manière spécifique avec le déterminant dans la région D2 d'IgE qui contient les domaines $C_H3$ et $C_H4$. On purifie les anticorps monoclonaux B9 provenant du liquide d'ascite de souris par une chromatographie sur protéine A-Sépharose (Ey et collaborateurs, Immunochemistry 15, 429-436, 1978) et on les élue dans la fraction à pH6 (fraction IgG1). La fraction contient 20% d'impuretés constituées d'IgG polyclonale sans activité anti-IgE.

On injecte les anticorps monoclonaux [100 µg dans 0,5 ml de NaCl(9 g/1) émulsifiés dans 0,5 ml d'adjuvant complet de Freund] par voie intradermique en des sites multiples toutes les deux semaines à des lapins blancs de Nouvelle-Zélande. On prélève du sang de ces animaux dix jours après la troisième injection et ensuite après chaque injection de rappel (Magnusson et collaborateurs, Clin. Allergy 11, 543-61, 1981). On sépare de ces anticorps anti_idiotypiques les anticorps dirigés contre des déterminants non-idiotypiques de protéines sériques et d'IgG de souris, par des passages successifs de l'antisérum anti-idiotypique sur des colonnes de Sépharose, auquel sont liées des protéines sériques et de l'IgG de souris par du glutaraldéhyde (Cambiaso et collaborateurs, Immunochemistry 12,273-278, 1975). On purifie alors les anticorps anti-idiotypiques IgG de lapin de l'antisérum absorbé au moyen d'une chromatographie sur protéine A-Sépharose (Magnusson et Masson, J. Allergy et Clin. Immun. 70, 326-336, 1982). On réduit ensuite les IgG purifiées en fragments $F(ab')_2$ par une digestion peptique (Magnusson et collaborateurs, Clin.Allergy 11, 543-561, 1981).

On couple 400 µg de ces fragments $F(ab')_2$ à 100 µl de latex carboxylé (100 g/l, Estapor K150, lot No. 501, Rhône-Poulenc, Courbevoie, France) par du carbodiimide (Magnusson et Masson ; J.Allergy and Clin. Immun. 70, 326-336, 1982). Avant utilisation, on a dilué le latex 160 fois. Les anticorps anti-idiotypiques IgG de lapin se trouvent donc sous la forme d'une suspension de latex recouvert de fragments $F(ab')_2$.

On dilue les sérums humains contenant l'IgE à doser dans un diluant à base de glycine contenant du NaCl ajusté à pH 9,2 avec du NaOH et additionné d'albumine de sérum bovin (BSA) à raison de 10 mg/ml, de sérum de lapin normal (NRS) à 10% et de fragments $F(ab')_2$ de lapin agglutinés par du glutaraldéhyde à raison de 0,6 mg/ml pour absorber les éventuels anticorps anti-fragments $F(ab')_2$. Cette solution formée d'un diluant et d'anticorps sera appelée ci-après NRS-BSA-F. Le diluant est formé en fait de 20 mg/ml de Dextran T500 (Pharmacia, Uppsala, Suède) dans de la glycine 0,1M contenant 50 mmoles d'acide éthylène diamine tétraacétique et 0,6 moles de NaCl, la solution étant ajustée à pH 9,2 avec du NaOH.

Poursuivre la réaction des anticorps monoclonaux B9 avec l'IgE, on préincube les échantillons de sérum humain avec les anticorps monoclonaux B9 pendant différentes périodes de temps et on mesure ensuite l'activité agglutinante résiduaire de ces anticorps monoclonaux B9 vis-à-vis du latex recouvert des anticorps anti-idiotypiques spécifiques des anticorps B9. On mesure l'agglutination en comptant le nombre de particules non
agglutinées dans un dispositif de comptage de particules optique relié à un système de prélèvement
d'échantillons et d'incubation automatisé (voir,
par exemple, Magnusson et collaborateurs, 1981).
On pourrait bien entendu également mesurer directement la quantité de latex agglutinée. Dans un
essai caractéristique, le dispositif aspire des
portions de 30 µl du mélange d'échantillon contenant
l' IgE et d'anticorps monoclonaux B9 et les injecte
dans un tube de réaction dans lequel 30 µl d'un
diluant et 30 µl d'anticorps anti-idiotypiques IgG
de lapin (sous la forme de fragments $F(ab')_2$) couplés à du latex ont été ajoutés séquentiellement
et automatiquement. Après 25 minutes d'incubation
sous une agitation tourbillonnante continue à 37°C,
on compte les particules non agglutinées. Dans le
cas où l'on dose l' IgE sans préincubation avec B9,
on ajoute B9 en même temps que le diluant. Pour
l'étude de corrélation, on a utilisé la méthode de
dosage d'IgE décrite précédemment par Magnusson et
Collaborateurs, 1981 .

Il est nécessaire dans le choix d'une concentration appropriée en anticorps monoclonaux B9
utilisés comme substance agglutinante, d'ajuster les
exigences contradictoires pour l'obtention d'une
sensibilité optimale et d'une précision satisfaisante
sur la gamme requise. La sensibilité optimale requiert
le choix d'une concentration en substance agglutinante
dans la partie la plus inclinée de la courbe
d'agglutination sigmoïdale. La précision

requiert , quant à elle,  une gamme plus large qui ne pourrait être obtenue que si l'agglutinat en l'absence d'inhibiteur  lie une proportion élevée de latex recouvert d'anticorps (Magnusson et Collaborateurs,1983). Sur la base de ces critères, on a choisi une concentration de 20 µg/litre d'anticorps monoclonaux B9, qui provoquent 75% d'agglutination.  On notera, à cet effet, que l'on choisira d'une manière générale une concentration en anticorps spécifiques dans l'échantillon liquide permettant d'obtenir, en l'absence de substance inhibitrice, un  degré d'agglutination d'au moins 50% avec les anticorps anti-idiotypiques.

L'inhibition exercée par l'IgE sur l'activité agglutinante des anticorps monoclonaux B9 vis-à-vis du latex recouvert d'anticorps anti-idiotipyque B9 a été examinée après différentes périodes de temps d'incubation entre l'IgE et  les anticorps monoclonaux B9 (voir figure 4). Comme on peut le voir sur cette figure 4, la hauteur de pic, représentant en fait le nombre de particules de latex non agglutinées en unités arbitraires, augmente avec l'élévation de la concentration en IgE dans l'échantillon.  C'est-à-dire qu'en fait l'addition de dose croissante d'IgE empêche proportionnellement l'activité agglutinante des anticorps monoclonaux B9 vis-à-vis du latex.  Les différences entre les quatre courbes montrent l'influence du temps de préincubation entre l'IgE et les anticorps monoclonaux B9 sur la sensibilité du dosage, la dernière courbe, sans période de préincubation, donnant la moins bonne sensibilité. En fait, la sensibilité s'élève de 40 UI/ml à 6 UI/ml d'IgE lorsque l'on accroît les temps de préincubation de O à 90 minutes. L'augmentation du temps de préincubation donne des courbes d'inhibition plus inclinées ( plus à pic) et, par conséquent, une meilleure précision.

On pense que le mécanisme d'inhibition et d'agglutination obtenu provient essentiellement de l'interaction de B9 avec l'IgE, qui inhibe l'agglutination de latex anti-B9 parce que les idiotopes de B9 sont masqués par la liaison d'IgE. Toutefois, étant donné que l'IgE est une molécule symétrique et qu'elle contient tous ses épitopes en double, on pense que la simple formation d'un réseau antigène - anticorps pourrait également participer au processus d'inhibition de l'agglutination entre les anticorps B9 et le latex anti-B9. Cette hypothèse a été examinée en utilisant du latex recouvert de fragments $F(ab')_2$ provenant d'anticorps anti-idiotypiques anti-C9 qui n'auraient pas été rendus spécifiques des idiotopes C9, c'est-à-dire qui n'auraient pas été purifiés. On a constaté que ce latex était agglutiné jusqu'à 60% par 5 µg/litre d'anticorps monoclonaux B9, ce qui montre que l'antisérum anti-C9 non absorbé a réagi avec des déterminants antigéniques qui n'étaient pas des idiotopes. Compte tenu de ce que la préparation d'anticorps monoclonaux B9 était contaminée par 20% d'IgG polyclonale, le latex a pu participer à l'activité agglutinante vis-à-vis du latex recouvert d'anti-IgG (voir ci-dessus). Malgré cette contamination par IgGI sans l'activité d'anticorps anti-IgE, l'addition de 40 UI/ml d'IgE a inhibé l'agglutination d'environ 10%. On peut donc conclure que la simple formation d'un complexe ou réseau d'antigène- anticorps entre les anticorps monoclonaux et leur antigène (molécule d'IgE) a contribué quelque peu au processus d'inhibition mais que le masquage de l'idiotype (B9) par la liaison de l'antigène (molécule d'IgE) joue certainement le rôle majeur.

Comme on le sait, l'utilisation de NRS-BSA-F (Collet-Cassart et Collaborateurs, 1983) comme diluant pour échantillons et témoins empêche l'agglutination non spécifique de latex recouvert de fragments $F(ab')_2$ d'IgG de lapin. Pour vérifier d'autres facteurs d'interférence éventuels, on a essayé 20 sérums contenant de faibles doses d'IgE (inférieures à 100 UI). Après une dilution de 1/10, on a essayé les sérums sans l'étape de préincubation entre les échantillons et les anticorps monoclonaux B9. Sous ces conditions, l'IgE dans ces échantillons se trouvait en-dessous de la limite de détection. Le coefficient de variation de la concentration de particules non agglutinées était de 2%, ce qui correspondait à 0,5 unité de hauteur de pic. Cette très faible valeur du coefficient de variation montre qu'il y a très peu d'interférences, et que l'on se trouve donc dans des conditions fiables. D'autre part, deux sérums avec des titres élevés de facteur rhumatoïde IgM et réagissant fortement avec les IgG de lapin, c'est-à-dire les anticorps anti-idiotypiques, n'agglutinaient pas le latex anti-B9, ce qui montre que l'utilisation de fragments $F(ab')_2$ au lieu d'IgG intacte à la surface des particules de latex prévient efficacement l'agglutination causée par le facteur rhumatoïde.

<u>Récupération analytique :</u>

Dix sérums de patients contenant 10 à 770 UI/ml d'IgE ont été additionnés de 2000 UI/ml d'IgE et l'IgE de ces sérums a été dosé après une dilution de 10 fois dans du NRS-BSA-F. La récupération analytique moyenne d'IgE était de 95,8% $\pm$ 8,7% (déviation standard) avec un coefficient de variation de 9,1%,ce qui montre qu'il n'y a pratiquement pas eu d'interférence entre l'IgE et le diluant NRS-BSA-F.

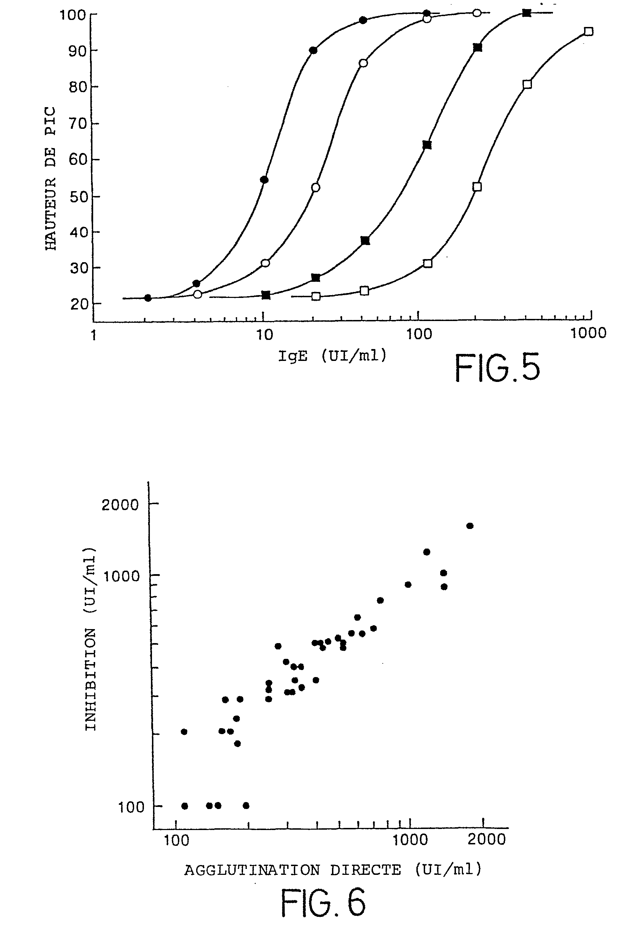

<u>Corrélation:</u> On a dosé de l'IgE par l'essai d'inhibition idiotypique de l'invention dans 41 sérums, qui avaient déjà été dosés par agglutination directe (latex recouvert d'anti-IgE agglutinant l'IgE). Le temps de préincubation des sérums contenant l'IgE avec B9 dans le dosage par inhibition était de 30 minutes. La corrélation entre les résultats de dosage d'IgE obtenus avec l'essai d'inhibition idiotypique de l'invention et l'essai d'agglutination directe est donnée par la figure 6. Comme on peut le voir sur cette figure, les résultats sont sensiblement similaires, se retrouvant pratiquement rassemblés dans une même zone. Le coefficient de corrélation entre les deux types de dosage est $r = 0,96$ avec une ligne de régression $y = 0,77 x + 97,4$ ($y$ = essai d'inhibition; $x$ = essai d'agglutination directe).

<u>Exemple 2. Dosage radioimmunologique d'un allergène (Dermatophagoïdes pteronyssinus ou DPT).</u>

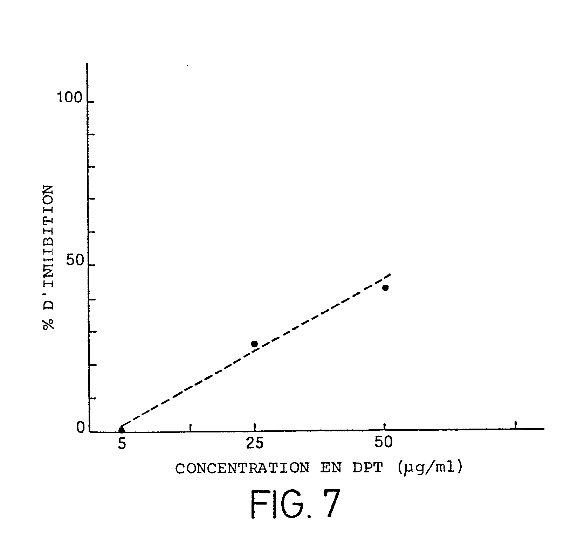

Le principe de ce dosage est représenté par la figure 4 des dessins annexés.Les puits d'une plaque de chlorure de polyvinyle(Flow Laboratories) sont recouverts d'immunoglobulines anti-D. pteronyssinus humaines (antisérum IgG) obtenues de patients allergiques. On incube, à cet effet, les plaques pendant 6 heures à une température de 37°C avec 100 ul, par puits, d'une solution saline (9 g/litre) tamponnée à pH 9,2 avec 50 mmoles de glycine contenant 10 ug/ml d'antisérum IgG. Après lavage avec la solution saline tamponnée, des dilutions en série de l'allergène (DPT) en solution saline (9 g/litre) tamponnée à pH 7,5 avec du Tris 0,05M et contenant 0,1% de sérum de veau foetal-(TSA-FCS) sont pipetées dans les puits, et la plaque est incubée pendant 2 heures à 37°C. Après quatre lavages avec de la solution saline (9 g/litre), on ajoute en portions de 50 ul à chaque puits des anticorps anti-idiotypiques IgG de lapin dilués dans du TSA-FCS à 2 mg/ml et on les incube pendant 8 heures à 37°C. On lave ensuite les plaques quatre fois avec de la solution saline (9 g/litre). On mesure ensuite la quantité d'anticorps anti-idiotypiques de lapin liés à l'antisérum IgG au moyen d'anticorps marqués dirigés contre les anticorps anti-idiotypiques IgG de lapin. Des portions de 50 µl d'anticorps de chèvre marqués au 125 I (50.000 cpm) et diluées dans du TSA-FCS sont pipetées dans les puits, incubées pendant 16 heures à 21°C et, après lavage, on compte la radioactivité dans un compteur de rayonnement gamma. Comme on peut le voir sur la figure 7, l'inhibition de la liaison des anticorps anti-idiotypiques de lapin est proportionnelle à la concentration en allergène. Les résultats sont calculés par la formule suivante:

$$(1 = \frac{\text{cpm de l'échantillon - cpm du contrôle A}}{\text{cpm du contrôle B - cpm du contrôle A}}) \times 100$$

le contrôle A représentant la radioactivité d'un puits sans anticorps anti-idiotypiques, c'est-à-dire la radioactivité non spécifique provenant de la liaison des anticorps de chèvre sur le support, le contrôle B la radioactivité d'un puits sans l'allergène (DPT), c'est-à-dire en fait la radioactivité maximale et cpm signifiant le nombre de coups par minute.

Exemple 3.

Dosage de thyroxine (T4) par comptage de particules (PACIA).

On prépare un antisérum de lapin dirigé contre l'idiotype d'anticorps anti-T4 monoclonaux (Hybritec, Liège, Belgique), comme décrit ci-dessus pour l'antisérum anti-idiotypique dirigé contre les anticorps anti-IgE monoclonaux. On utilise ensuite l'antisérum anti-idiotypique pour le dosage de T4 en utilisant soit du latex recouvert des anticorps anti-idiotypiques , soit du latex recouvert des anticorps monoclonaux.

Dans le premier procédé ( qui suit scrupuleusement les détails donnés pour la détermination spécifique d'IgE ; voir exemple 1), on recouvre le latex de fragments $F(ab')_2$ de l'IgG anti-idiotypique , comme décrit dans le dosage d'IgE de l'exemple 1. On mesure alors l'activité agglutinante des anticorps monoclonaux. On constate qu'une concentration de 10 µg/litre en anticorps monoclonaux, provoquant une agglutination de 80%, donne la sensibilité la plus élevée dans l'essai d'inhibition. L'inhibition

exercée par la T4 solubilisée en solution saline (9 gr/litre) sur l'activité agglutinante des anticorps anti-T4 monoclonaux est testée après différents périodes d'incubation entre T4 et les anticorps anti-T4 monoclonaux. La sensibilité s'accroît de 40 à 20 ng/litre en augmentant la période d'incubation de 0 à 30 minutes, la sensibilité étant définie par 3 unités de hauteur de pic. Des périodes d'incubation plus longues que 30 minutes n'améliorent pas la sensibilité. La gamme dynamique s'étend de 20 à 320 ng/ml.

Dans le second procédé, on recouvre le latex d'anticorps anti-T4 monoclonaux et on utilise l'antisérum anti-idiotypique comme substance agglutinante. L'activité agglutinante de cet antisérum dilué à 1/4000 est accrue par l'addition d'un sérum rhumatoïde dilué à 1/50, comme décrit dans le brevet des Etats-Unis d'Amérique n° 4.427.781. On a choisi ce sérum rhumatoïde sur la base de sa forte activité agglutinante vis-à-vis du latex recouvert d'IgG de lapin et de son manque d'activité agglutinante vis-à-vis du latex recouvert d'IgG de souris. L'inhibition exercée par T4 solubilisée en solution saline (9 gr/litre) sur l'agglutination est testée après différents temps d'incubation entre T4 et le latex anti-T4 avant l'addition de l'anti-idiotype de lapin additionné du facteur rhumatoïde . La sensibilité s'accroît de 25 ng/ml à 1 ng/ml lorsque l'on augmente les temps de préincubation de 0 à 30 minutes. Des incubations plus longues n'améliorent pas la sensibilité au-delà de 1 ng/ml. La gamme dynamique s'étend de 1 ng/ml   100 ng/ml.

Il doit être entendu que la présente invention n'est en aucune façon limitée aux formes de réalisation ci-dessus et que bien des modifications peuvent y être apportées sans sortir du cadre du présent brevet.

REVENDICATIONS.

1. Procédé de dosage immunologique d'une substance dans un échantillon liquide, tel qu'un fluide biologique, ladite substance comportant au moins un déterminant important pour l'identification de celle-ci, caractérisé en ce qu'il comprend la mise en réaction dudit échantillon liquide contenant la substance à doser, d'anticorps dirigés contre ladite substance, spécifiques dudit déterminant et d'anticorps anti-idiotypiques dirigés contre les anticorps spécifiques et la détermination de la quantité de la substance dans l'échantillon liquide en mesurant le degré d'inhibition qu'entraîne cette substance sur la réaction de liaison entre les anticorps spécifiques et anti-idiotypiques susdits, la quantité de cette substance étant proportionnelle au degré d'inhibition obtenu sur cette réaction de liaison.

2. Procédé suivant la revendication 1, caractérisé en ce que soit les anticorps anti-idiotypiques, soit les anticorps spécifiques sont fixés à des particules finement divisées et en ce que l'on détermine la quantité de substance dans l'échantillon liquide, après réaction de cette substance et des anticorps spécifiques et anti-idiotypiques précités, en fonction du degré d'agglutination résiduaire ou de non-agglutination de ces particules finement divisées·

3. Procédé suivant la revendication 2, caractérisé en ce que l'on utilise comme particules finement divisées des particules de latex ou des globules rouges.

4. Procédé suivant l'une ou l'autre des revendications 2 et 3, caractérisé en ce que la concentration en anticorps spécifiques dans l'échantillon liquide est telle qu'elle permet d'obtenir, en l'absence de substance inhibitrice, un degré d'agglutination d'au moins 50% avec les anticorps anti-idiotypiques.

5. Procédé suivant la revendication 4, caractérisé en ce que la concentration en anticorps spécifiques précitée permet d'obtenir un degré d'agglutination de l'ordre de 75 à 80% avec les anticorps anti-idiotypiques.

6. Procédé suivant l'une quelconque des revendications 2 à 5, caractérisé en ce que l'on accroît l'activité agglutinante des anticorps anti-idiotypiques par l'addition d'un agent susceptible d'accroître une telle activité.

7. Procédé suivant la revendication 6, caractérisé en ce que l'agent susceptible d'accroître l'activité agglutinante des anticorps anti-idiotypiques est choisi dans le groupe comprenant le facteur rhumatoïde, le facteur Clq du complément et le polyéthylène glycol.

8. Procédé suivant l'une quelconque des revendications 2 à 7, caractérisé en ce que dans le cas où les anticorps anti-idiotypiques sont fixés à des particules finement divisées, on préincube l'échantillon liquide contenant la substance à doser avec les anticorps spécifiques sous agitation à une température de l'ordre de 37°C pendant une durée de l'ordre de 1 minute à 18 heures.

9. Procédé suivant la revendication 1, caractérisé en ce que les anticorps anti-idiotypiques sont marqués au moyen d'une substance active choisie dans le groupe comprenant les radioisotopes, les enzymes, les fluorochromes et les particules colloïdales et les anticorps spécifiques couplés à une phase solide de manière à les insolubiliser, et en ce que l'on ajoute l'échantillon liquide contenant ladite substance à doser aux anticorps insolubilisés, on laisse réagir ces anticorps insolubilisés avec ladite substance, on ajoute à l'échantillon ainsi obtenu les anticorps anti-idiotypiques marqués, on laisse réagir ces anticorps anti-idiotypiques marqués, on sépare de la phase solide les anticorps anti-idiotypiques marqués non liés aux anticorps insolubilisés et on détermine la quantité de substance à doser dans l'échantillon liquide en fonction de l'activité résiduaire des anticorps anti-idiotypiques marqués liés à la phase solide.

10. Procédé suivant la revendication 1, caractérisé en ce que les anticorps spécifiques sont marqués au moyen d'une substance active choisie dans le groupe comprenant les radioisotopes, les enzymes, les fluorochromes et les particules colloïdales et les anticorps anti-idiotypiques couplés à une phase solide de manière à les insolubiliser et en ce que l'on ajoute l'échantillon liquide contenant la substance à doser aux anticorps insolubilisés, on laisse réagir ces anticorps insolubilisés avec ladite substance, on ajoute à l'échantillon ainsi obtenu les anticorps spécifiques marqués, on laisse réagir ces anticorps spécifiques marqués, on sépare de la phase solide les anticorps spécifiques marqués non liés aux anticorps insolubilisés et on détermine la quantité de substance à doser dans l'échantillon liquide en fonction de l'activité résiduaire des anticorps spécifiques marqués liés à la phase solide.

11. Procédé suivant la revendication 1, caractérisé en ce que les anticorps spécifiques sont couplés à une phase solide de manière à les insolubiliser et en ce que l'on ajoute l'échantillon liquide contenant la substance à doser aux anticorps insolubilisés, on laisse réagir ces anticorps insolublisés avec ladite substance, on ajoute à l'échantillon ainsi obtenu les anticorps anti-idiotypiques, on laisse réagir ces anticorps anti-idiotypiques, on sépare de la phase solide les anticorps anti-idiotypiques non liés aux anticorps insolubilisés, on ajoute à la phase solide des anticorps dirigés contre les anticorps anti-idiotypiques, marqués au moyen d'une substance active et choisie dans le groupe comprenant les radioisotopes, les enzymes, les fluorochromes et les particules colloïdales, on laisse réagir ces anticorps marqués, on sépare de la phase solide les anticorps marqués non liés aux anticorps anti-idiotypiques et on détermine la quantité de substance à doser dans l'échantillon liquide en fonction de l'activité résiduaire des anticorps marqués liés à la phase solide.

12. Procédé suivant l'une quelconque des revendications 9 à 11, caractérisé en ce que la phase solide est constituée d'un support polymère, tel que microplaques de chlorure de polyvinyle, billes d'agarose, disques de papier ou cellulosiques.

13. Procédé suivant l'une quelconque des revendications 9 à 12, caractérisé en ce que la séparation des anticorps non liés de la phase solide se fait par lavage dans une solution saline.

14. Procédé suivant l'une quelconque des revendications 9 à 13, caractérisé en ce que l'on préincube l'échantillon liquide contenant la substance à doser avec les anticorps marqués sous agitation à une température de l'ordre de 37°C pendant une durée de l'ordre de 1 minutes à 18 heures.

15. Procédé suivant l'une quelconque des revendications 1 à 14, caractérisé en ce que la mise en réaction précitée comprend l'incubation de l'échantillon liquide contenant la substance à doser, des anticorps spécifiques et des anticorps anti-idiotypiques sous agitation à une température denviron 37°C pendant une durée de l'ordre de 15 minutes à 24 heures.

16. Procédé suivant l'une quelconque des revendications 1 à 15, caractérisé en ce que la substance à doser est de structure moléculaire simple, comportant un seul déterminant important pour son identification.

17. Procédé suivant la revendication 16, caractérisé en ce que la substance simple est la triiodothyronine (T3), les anticorps spécifiques des anticorps anti-T3 monoclonaux de souris et les anticorps anti-idiotypiques des anticorps anti-idiotypiques IgG de lapin dirigés contre des anticorps monoclonaux de souris.

18. Procédé suivant la revendication 16, caractérisé en ce que la substance simple est la thyroxine, les anticorps spécifiques des anticorps anti-T4 monoclonaux de souris et les anticorps anti-idiotypiques des anticorps anti-idiotypiques IgG de lapin dirigés contre les anticorps monoclonaux de souris.

19. Procédé suivant l'une quelconque des revendications 1 à 15, caractérisé en ce que la substance à doser est de structure moléculaire complexe, comportant un ou plusieurs déterminants importants pour son identification.

20. Procédé suivant la revendication 19, caractérisé en ce que la substance complexe est l'antigène carcinoembryonnaire (CEA), les anticorps spécifiques des anticorps anti-CEA monoclonaux de souris et les anticorps anti-idiotypiques des anticorps anti-idiotypiques IgG de lapin dirigés contre les anticorps monoclonaux de souris.

21. Procédé suivant la revendication 19, caractérisé en ce que la substance complexe est l'immunoglobuline IgE, les anticorps spécifiques des anticorps anti-IgE monoclonaux de souris et les anticorps anti-idiotypiques des anticorps anti-idiotypiques IgG de lapin dirigés contre les anticorps monoclonaux de souris.

22. Procédé suivant la revendication 21, caractérisé en ce que les anticorps anti-idiotypiques, lorsqu'ils sont liés à des particules de latex, sont sous la forme d'une suspension de latex recouvert de fragments $F(ab')_2$.

23. Procédé suivant la revendication 19, caractérisé en ce que la substance complexe est un allergène, les anticorps spécifiques des anticorps anti-allergène humains, les anticorps anti-idiotypiques des anticorps anti-idiotypiques IgG de lapin dirigés contre les anticorps humains et en ce que l'on utilise comme anticorps marqués des anticorps dirigés contre les IgG de lapin, marqués au 125 I.

FIG.1

← SECOND ANTICORPS MARQUE

← ANTIGENE

← PREMIER ANTICORPS

PHASE SOLIDE

FIG.2

← ANTICORPS ANTI-IDIOTYPIQUE MARQUE

← ANTIGENE

← ANTICORPS SPECIFIQUE

PHASE SOLIDE

FIG.3

← ANTICORPS SPECIFIQUE MARQUE

← ANTIGENE

← ANTICORPS ANTI-IDIOTYPIQUE

PHASE SOLIDE

FIG. 4

← ANTICORPS MARQUE

ANTIGENE →

← ANTICORPS ANTI-IDIOTYPIQUE

← ANTICORPS SPECIFIQUE

PHASE SOLIDE

FIG.5

FIG. 6

FIG. 7

Office européen
des brevets

RAPPORT DE RECHERCHE EUROPEENNE

0170302
Numéro de la demande

EP  85 20 0746

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| P,X | EP-A-0 119 629   (NEW ENGLAND NUCLEAR CORP.)<br><br>* en entier *<br><br>--- | 1-6,9-19,21, 23 | G 01 N  33/543 //<br>G 01 N  33/546<br>G 01 N  33/577 |
| A | CHEMICAL ABSTRACTS, vol. 100, no. 1, 2 janvier 1984, page 163, colonne 1, résumé no. 4534h, Columbus, Ohio, US; J.H. NOSEWORTHY et al.: "Cell receptors for the mammalian reovirus. I. Syngeneic monoclonal anti-idiotypic antibody identifies a cell surface receptor for reovirus", & J. IMMUNOL. 1983, 131(5), 2533-2538<br><br>--- | 1 | |
| A | CHEMICAL ABSTRACTS, vol. 100, no. 1, 2 janvier 1984, page 163, colonne 1, résumé no. 4535j, Columbus, Ohio, US; J.H. NOSEWORTHY et al.: "Cell receptors for the mammalian reovirus. II. Monoclonal anti-idiotypic antibody blocks viral binding to cells", & J. IMMUNOL. 1983, 131(5), 25<br><br>--- | 1 | DOMAINES TECHNIQUES RECHERCHES (Int Cl 4)<br><br>G 01 N  33/00 |
| A | US-A-4 184 849   (C.L. CAMBIASO et al.)<br><br>----- | | |

Le present rapport de recherche a éte etabli pour toutes les revendications

| Lieu de la recherche | Date d'achèvement de la recherche | Examinateur |
|---|---|---|
| BERLIN | 23-09-1985 | GREEN C.H. |